# EUROPEAN PATENT APPLICATION

(11) **EP 4 501 354 A1**
(43) Date of publication of application: **05.02.2025**
(21) Application number: 23781043.7
(22) Date of filing: 31.03.2023
(51) Int. Cl.: A61K 39/395, A61P 1/16, A61P 43/00, C07K 16/40, C12N 15/13

(54) **COMPOSITION AND METHOD FOR INHIBITING FIBROSIS**

(30) Priority: 31.03.2022 JP 2022061154
(71) Applicant: Pharma Foods International Co., Ltd., Kyoto 615-8245 (JP)
(72) Inventor: SHOYA, Yuji, Kyoto-shi, Kyoto 615-8245 (JP); SAITO, Kenji, Kyoto-shi, Kyoto 615-8245 (JP); SAKATA, Tomoko, Kyoto-shi, Kyoto 615-8245 (JP); SHIGEMITSU, Takanari, Kyoto-shi, Kyoto 615-8245 (JP); MONWAN, Warunthorn, Kyoto-shi, Kyoto 615-8245 (JP); SHIMADA, Kana, Kyoto-shi, Kyoto 615-8245 (JP); SOMEDA, Masataka, Kyoto-shi, Kyoto 615-8245 (JP)
(74) Representative: Gulde & Partner
(86) International application number: PCT/JP2023/013490
(87) International publication number: WO 2023/191035

(57) **Abstract**

Provided is a novel composition or method for inhibiting fibrosis. This composition for inhibiting fibrosis comprises an antibody that specifically binds to PAD2 and is used. In addition, a pharmaceutical composition for treating fibrotic disease comprises an antibody that specifically binds to PAD2 and a pharmaceutically acceptable carrier, and may be used. Further, a kit for inhibiting fibrosis comprises an antibody that specifically binds to PAD2 and may be used.

## Description

### Technical Field

The technical field of the present invention relates to a composition or method for inhibiting fibrosis.

### Background Art

Fibrosis is known as a phenomenon caused by excessive accumulation of connective tissue in tissues and is said to be responsible for up to 45% of all deaths in industrialized countries (Non-Patent Literature 1).

Several reports have recently been published on the causes of fibrosis. Non-Patent Literature 2 (2019) describes ER stress increase and cell death induction due to impaired secretion of SFTPA1 protein as a cause of fibrosis.

Non-Patent Literature 3 (2020) describes NEAT1 degradation and cell death induction due to RBM7 protein as a cause of fibrosis.

Non-Patent Literature 4 (2021) describes fibroblast infiltration mediated and caused by citrullinated vimentin as a cause of fibrosis.

### Citation List

### Non-Patent Literature

Non-Patent Literature 1: "FIBROSIS: FROM MECHANISMS TO MEDICINES" Henderson et al., Nature, 2020 Nov; 587(7835): 555-566.
Non-Patent Literature 2: "A homozygous SFTPA1 mutation drives necroptosis of type II alveolar epithelial cells in patients with idiopathic pulmonary fibrosis" Takezaki et al., J Exp Med., 2019 Dec 2;216(12):2724-2735.
Non-Patent Literature 3: "Dysregulated Expression of the Nuclear Exosome Targeting Complex Component Rbm7 in Nonhematopoietic Cells Licenses the Development of Fibrosis" Fukushima et al., Immunity, 2020 Mar 17;52(3):542-556.e13.
Non-Patent Literature 4: "Citrullinated vimentin mediates development and progression of lung fibrosis" Li et al., Sci Transl Med., 2021 Mar 17;13(585):eaba2927.

### Summary of Invention

### Technical Problem

Although various findings have been accumulated, many of the causes of fibrosis still remain unclear. Thus, conventional treatment strategies alone have not been sufficient.

Meanwhile, the present inventors conducted intensive research and, as a result, have found that administration of an anti-PAD2 antibody to fibrosis model mice can inhibit fibrosis.

The results of this study were unexpected based on the prior art. For example, the above Non-Patent Literatures 1 to 3 are silent on anti-PAD2 antibodies or PAD2. Non-Patent Literature 4 states "Cd/CB (cadmium/carbon black) induced secretion of citrullinated vimentin in an Akt1- and PAD2-dependent manner" (see the Abstract). However, according to Non-Patent Literature 4, citrullinated vimentin is produced (and subsequently secreted) by the "intracellular" citrullination of vimentin. On the other hand, since the anti-PAD2 antibody is considered to act "extracellularly", those skilled in the art cannot recognize that the anti-PAD2 antibody affects vimentin citrullination intracellularly. Therefore, it is difficult to predict that fibrosis is inhibited by the anti-PAD2 antibody from the literature on intracellular citrullination mechanisms as in Non-Patent Literature 4.

### Solution to Problem

An aspect of the present invention provides a composition for inhibiting fibrosis, comprising an antibody that specifically binds to PAD2. This composition can be used to inhibit fibrosis.

Another aspect of the present invention provides a pharmaceutical composition for treating fibrotic disease, comprising an antibody that specifically binds to PAD2, and a pharmaceutically acceptable carrier. This pharmaceutical composition can be used to treat fibrotic disease.

Another aspect of the present invention provides a method of inhibiting or treating fibrosis, comprising the step of administering an anti-PAD2 antibody to a subject. This method can be used to inhibit or treat fibrosis.

### Brief Description of Drawings

[Figure 1] Fig. 1 is a table showing the results of measuring affinity of each anti-PAD2 antibody for PAD2.
[Figure 2] Fig. 2 is a graph showing the results of measuring the ability of each anti-PAD2 antibody to inhibit PAD2 activity.
[Figure 3] Fig. 3 is a graph showing the results of measuring a fibrosis percentage after each anti-PAD2 antibody was administered to a fibrosis model.
[Figure 4] Fig. 4 are images showing the results of measuring the ability of each anti-PAD2 antibody to inhibit PAD2 activity in a fibrosis model.

### Description of Embodiments

Hereinafter, embodiments of the present invention will be described in detail. Note that repeated descriptions of the same content are omitted, if appropriate, so as to avoid redundancy.

An embodiment of the present invention provides a novel composition for inhibiting fibrosis. This composition is, for example, a composition containing an antibody that specifically binds to PAD2. Such a composition can be used to inhibit fibrosis as demonstrated in the Examples below.

In one embodiment of the present invention, PAD2 includes a protein called Peptidylarginine deiminase 2, Peptidyl arginine deiminase type-2, or Protein-arginine deiminase type-2. These terms can be used interchangeably. Details of the amino acid sequence of PAD2 can be obtained from, for instance, NCBI or UniProt website. The primary accession number of PAD2 listed in UniProt is, for example, Q9Y2J8. The amino acid sequence of human PAD2 is, for instance, SEQ ID NO: 1. If PAD2 has PAD2 activity, the biological origin is not limited. The PAD2 activity involves, for instance, citrullination activity. Examples of the citrullination activity include an activity to catalyze the citrullination of arginine side chains in a substrate. The PAD2 activity may be evaluated by incubating a solution containing both PAD2 and a substrate and then colorimetrically quantifying citrulline residues. Examples of the substrate include a compound with an arginine side chain(s) (e.g., a protein (e.g., histone), peptide, or low-molecular-weight compound (e.g., BAEE)). The colorimetric quantification may be performed using a mixture containing 2,3-butanedione monoxime and thiosemicarbazide. Examples of PAD2 include PAD2 derived from a human, monkey, mouse, rat, dog, or cat.

In one embodiment of the present invention, the anti-PAD2 antibody can inhibit fibrosis. Examples of the fibrosis include a phenomenon caused by the excessive accumulation of connective tissue in tissues. Tissue stiffening associated with fibrosis may occur, for example, when connective tissues composed of collagen and other components are increased and replaced by normal tissues. Examples of the fibrosis include fibrosis occurring in tissues such as the liver, lung, kidney, heart, pancreas, bone marrow, or skin. In one embodiment of the present invention, the fibrotic disease includes a disease accompanied by tissue fibrosis.

In one embodiment of the present invention, the anti-PAD2 antibody includes an antibody with an ability to inhibit PAD2 activity. Examples of the activity inhibition include citrullination activity inhibition. In one embodiment of the present invention, the anti-PAD2 antibody includes an antibody with an ability to inhibit fibrosis. In one embodiment of the present invention, the anti-PAD2 antibody includes an antibody with PAD2-neutralizing activity (neutralizing antibody), an antibody with an ability to inhibit PAD2 function, or an antibody with an ability to reduce the amount or percentage of fibrosis tissue in an individual or tissue. Examples of the neutralizing antibody include an antibody that can inhibit PAD2 activity. In one embodiment of the present invention, examples of the tissue include the liver, lung, kidney, heart, pancreas, bone marrow, or skin. As used herein, the antibodies that inhibit PAD2 activity include an antibody with a function of inhibiting PAD2 activity, or an antibody with an ability to inhibit PAD2 activity. As used herein, the antibodies that inhibit fibrosis include an antibody with a function of inhibiting fibrosis, or an antibody with an ability to inhibit fibrosis.

In one embodiment of the present invention, the anti-PAD2 antibody may be produced, for example, by immunizing mammals or birds with PAD2 and then collecting and purifying the antibody. The anti-PAD2 antibody may be produced using various techniques and modeling technology known in the art, for example, in "Development of therapeutic antibodies for the treatment of diseases, Lu et al., J Biomed Sci., volume 27, Article number: 1 (2020 Jan 2)" or "Modeling Immunity with Rosetta: Methods for Antibody and Antigen Design, Schoeder et al., Biochemistry, 2021 Mar 23; 60(11): 825-846". The anti-PAD2 antibody may be produced through the steps of immunizing a mammal or bird with, for instance, a peptide consisting of the amino acid sequence set forth in SEQ ID NO: 2 and collecting and purifying the antibody. For example, full-length PAD2 or a fragment thereof may be used for PAD2 utilized as the antigen. The anti-PAD2 antibody may be produced through the step of selecting an anti-PAD2 antibody that show binding affinity to a wild-type PAD2, but not to a PAD2 mutated at positions 341 to 357 (e.g., a deletion mutant). The anti-PAD2 antibody may be produced through the step of selecting the antibody based on their strength of binding to PAD2. The anti-PAD2 antibody may be produced through the step of selecting the antibody with PAD2-neutralizing activity. The PAD2-neutralizing activity may be evaluated by the steps of (i) preparing and incubating a mixture containing PAD2 and an anti-PAD2 antibody, (ii) adding and incubating a substrate of PAD2 in the mixture, or (iii) colorimetrically quantifying citrulline residues of the substrate. At this time, the PAD2 neutralizing activity is indicated by a decrease in the amount of citrulline residues during the anti-PAD2 antibody treatment when compared to that during the non-anti-PAD2 antibody or negative control treatment. The anti-PAD2 antibody may be produced through the step of selecting the antibody with an effect of inhibiting fibrosis.

In one embodiment of the present invention, the anti-PAD2 antibody may be used, for example, PK1-16, CK1-10, or CK1-14 as described below; S4, S10, S24, S47, S108, S113, S170, or S309 as described in WO2019/244934 A1; #2, #6, or #34 as described in WO2014/086365 A1; mSol1, mSol2, mSol3, or mSol4 as described in WO2016/155745 A1; Ab16478 (Abcam) as described in Zhou et al., Front Immunol., 2017 Sep 25;8:1200; DN6, DN18, DN31, or DN34 as described in Damgaard et al., J Immunol Method, 2014 Mar;405:15-22; 66386-1-Ig (Proteintech) as described in Kim et al., Cell Mol Life Sci. 2022 Feb 26;79(3):155; or any known anti-PAD2 antibody, such as 0G8 (Creative Diagnostics), 4D4 (Sigma), clone 9F7 (Cayman Chemical), ARG40489 (Arigo), MBS839991 (MyBioSource), AF7257 (Bio-Techne), A11711 (ABclonal, Inc.), or A2322 (BioVision Inc.).

In one embodiment of the present invention, the anti-PAD2 antibody include an antibody that has binding affinity to a peptide consisting of the amino acid sequence represented by SEQ ID NO: 2 or positions 341-357 of PAD2. This antibody may have binding affinity to bind to other amino acid residue(s) within PAD2 as long as the antibody has binding affinity to a peptide consisting of the amino acid sequence represented by SEQ ID NO: 2 or positions 341-357 of PAD2. The anti-PAD2 antibody may be, for instance, an anti-PAD2 antibody that has binding affinity to a wild-type PAD2, but has no binding affinity to a PAD2 mutated at positions 341 to 357 (e.g., a deletion mutant). In one embodiment of the present invention, the antibody that specifically binds to a specific site may be an antibody that recognizes the specific site. In one embodiment of the present invention, the wording "have binding affinity to" includes "have substantially binding affinity to" or the case of "have significantly binding affinity to". Examples of an antibody that has binding affinity to an antigen (e.g., a peptide consisting of the amino acid sequence represented by SEQ ID NO: 2) may include an antibody with a K_{D} (M) toward the antigen of 9.0 × 10⁻⁸ or less (including, for example, the range of K_{D} (M) toward the antigen described below). The K_{D} (M) may be a value measured by SPR. In one embodiment of the present invention, the wording "have no binding affinity to" includes "have no substantially binding affinity to" or the case of "have no significantly binding affinity to". Alternatively, the case where EC₅₀ for an analyte is 2, 10, 100, or 10000 times larger than EC₅₀ for the wild-type counterpart may be evaluated as "have no binding affinity to". At this time, the case where EC₅₀ is "Not Determined" may be evaluated as "have no binding affinity to". Alternatively, the case where binding to an analyte is 50, 30, 10, 5, or 1% or less than binding to the wild-type counterpart may be evaluated as "have no binding affinity to".

In one embodiment of the present invention, the anti-PAD2 antibody includes the form of monoclonal antibody. The monoclonal antibody could act on PAD2 more efficiently than a polyclonal antibody.

In one embodiment of the present invention, the anti-PAD2 antibody includes a form of antibody fragment having PAD2-binding activity (hereinafter, sometimes referred to as an "antigen-binding fragment"). Examples of the antigen-binding fragment include an antigen-binding fragment having any of the CDR sets in (a)-(c) below and having PAD2-binding activity.

In one embodiment of the present invention, the anti-PAD2 antibody may have a K_{D} (M) of equal to or less than or between any two values of, for instance, 9.0 × 10⁻⁸, 5.0 × 10⁻⁸, 1.0 × 10⁻⁸, 9.0 × 10⁻⁹, 5.0 × 10⁻⁹, 1.0 × 10⁻⁹ , 9.0 × 10⁻¹⁰, 5.0 × 10⁻¹⁰, 1.0 × 10⁻¹⁰ or less. The K_{D} (M) may be a value measured by SPR.

In one embodiment of the present invention, the antibody class of the anti-PAD2 antibody is not particularly limited and may be, for instance, IgM, IgD, IgG, IgA, IgE, or IgY. In addition, a subclass of the antibody is not particularly limited and may be, for instance, IgG1, IgG2, IgG3, IgG4, IgA1, or IgA2.

In one embodiment of the present invention, the anti-PAD2 antibody includes an antibody that binds extracellularly to PAD2. The extracellular may be extracellular *in vivo.* In one embodiment of the present invention, the anti-PAD2 antibody may be an antibody that binds or inhibits only PAD2 of the PAD1-6 family. In one embodiment of the present invention, the anti-PAD2 antibody may be used for single therapy.

In one embodiment of the present invention, the anti-PAD2 antibody may be an antibody that binds to a wild-type PAD2 or a PAD2 mutant. Examples of the PAD2 mutant include those having an SNP(s), etc., that is caused by variation in individual DNA sequences.

In one embodiment of the present invention, the anti-PAD2 antibody includes an antibody that has binding affinity to PAD2. The antibody includes a molecule or population thereof that can specifically bind to a specific epitope on an antigen. In addition, the antibody may be a polyclonal or monoclonal antibody. The antibody may be present in various forms, and examples include at least one form selected from the group consisting of full-length antibody (antibodies with Fab and Fc regions), Fv antibody, Fab antibody, F(ab')₂ antibody, Fab' antibody, diabody, single-chain antibody (e.g., scFv), dsFv, multispecific antibody (e.g., bispecific antibody), antigen binding peptides or polypeptides, chimeric antibody, mouse antibody, chicken antibody, humanized antibody, human antibody, and their equivalents (or equivalents). In addition, the antibody may include a modified antibody or an unmodified antibody. The modified antibody may be an antibody linked to various molecule such as polyethylene glycol. The modified antibody may be obtained by chemically modifying an antibody using a known procedure. The antibody may also be a fusion protein. This fusion protein may have a polypeptide or oligopeptide (e.g., a His tag) linked to the N or C terminal of the antibody. Also, the fusion protein may be a product of fusing a part(s) of mouse, chicken, or human antibody sequence. The antibody may also be a conjugate antibody (e.g., antibody-drug conjugate). The drug may be, for example, a cytotoxic or anticancer agent. The antibody may also be a function-modified antibody. Such a modified antibody, fusion protein, conjugate, or function-modified antibody is also included in a form of the antibody. The amino acid sequence, class, or subclass of the antibody may be derived from, for instance, a human, a non-human mammal (e.g., a rat, mouse, rabbit, cow, monkey), or a bird (e.g., a chicken). In addition, examples of the antibody include an isolated antibody, a purified antibody, or a recombinant antibody. Further, the antibody may be used, for instance, *in vitro* or *in vivo.*

In one embodiment of the present invention, the polyclonal antibody may be generated by administering an immunogen containing an antigen of interest to, for instance, a mammal (e.g., a rat, mouse, rabbit, cow, monkey) or a bird (e.g., a chicken). The immunogen may be administered by injecting at least one immunizing agent or adjuvant. The adjuvant may be used to augment immune response and may contain Freund's adjuvant (complete or incomplete), mineral gel (e.g., aluminum hydroxide), or a surfactant (e.g., lysolecithin). The immunization protocol has been known in the art and may be implemented by any procedure in which immune response is induced in accordance with a host organism chosen (Protein Experiment handbook, YODOSHA CO., LTD., (2003): 86-91).

In one embodiment of the present invention, the monoclonal antibody includes an antibody in the case where individual antibodies constituting a population substantially react with the identical epitope. Alternatively, the monoclonal antibody may be an antibody in the case where individual antibodies constituting a population are substantially the same (provided that naturally occurring mutations are permitted). The method for preparing a monoclonal antibody is not particularly limited, and the monoclonal antibody may be prepared by substantially the same method as the hybridoma method described in, for instance, "Kohler G, Milstein C., Nature, 1975 Aug 7; 256(5517): 495-497". Alternatively, the monoclonal antibody may be prepared by substantially the same method as the recombinant method disclosed in U.S. Patent No. 4816567. Alternatively, the monoclonal antibody may be isolated from a phage antibody library by using substantially the same method as the technology described in "Clackson et al., Nature, 1991 Aug 15; 352(6336): 624-628" or "Marks et al., J Mol Biol., 1991 Dec 5; 222(3): 581-597". Alternatively, the method described in "Protein Experiment handbook, YODOSHA CO., LTD. (2003): 92-96" may be used for the preparation.

In one embodiment of the present invention, the chimeric antibody may be, for instance, a product of linking a variable region of an antibody and a constant region of an antibody from different species of organisms and can be prepared by gene recombinant technology. Examples include a non-human/human-derived chimeric antibody (e.g., a mouse/human chimeric antibody, a chicken/human chimeric antibody, or a chicken/mouse chimeric antibody). The mouse/human chimeric antibody can be produced by, for instance, the method described in "Roguska et al., Proc Natl Acad Sci USA, 1994 Feb 1; 91(3): 969-973". In a basic method for producing the mouse/human chimeric antibody, a mouse leader sequence and variable region sequences present in a cloned cDNA are linked to a sequence encoding a human antibody constant region already present in a mammalian expression vector. Alternatively, a mouse leader sequence and variable region sequences present in a cloned cDNA may be linked to a sequence encoding a human antibody constant region, and this may be integrated into a mammalian expression vector. A fragment of human antibody constant region may be a H-chain constant region or a L-chain constant region of any human antibody. For example, Cγ1, Cγ2, Cγ3, or Cγ4 for the human H-chain, and Cλ or Cκ for the L-chain.

In one embodiment of the present invention, the humanized antibody includes an antibody that has, for instance, at least one non-human CDR and human immunoglobulin-derived framework region, and a human immunoglobulin-derived constant region, and can bind to a desired antigen. Various techniques known in the art may be used to humanize the antibody as described in, for instance, "Safdari et al., Biotechnol Genet Eng Rev., 2013; 29: 175-86".

In one embodiment of the present invention, the human antibody refers to an antibody in which variable and constant regions of a heavy chain and variable and constant regions of a light chain of an antibody, for instance, are derived from a gene encoding a human immunoglobulin. Various techniques known in the art may be used to prepare the human antibody as described in, for instance, "Duvall et al., MAbs., 2011 Mar-Apr; 3(2): 203-208".

In one embodiment of the present invention, the Fv antibody refers to an antibody including an antigen-recognition site. This region contains a dimer of one heavy chain variable region and one light chain variable region that are noncovalently bonded. In this structure, three CDRs of each variable domain interact with each other to be able to form an antigen-binding site on a surface of the VH-VL dimer.

In one embodiment of the present invention, the Fab antibody refers to an antibody obtained by, for instance, treating an antibody containing Fab and Fc regions with a protease papain to give fragments, in which about a half of the H chain on the N-terminal side and the whole L chain are bonded via a disulfide bond. The Fab can be obtained by, for instance, digesting, with a protease papain, an anti-PAD2 antibody containing Fab and Fc regions in the above embodiment of the present invention.

In one embodiment of the present invention, the F(ab')₂ antibody refers to an antibody obtained by, for instance, treating an antibody containing Fab and Fc regions with a protease pepsin to give fragments, in which two Fab comparable portions are included. The F(ab')₂ can be obtained by, for instance, digesting, with a protease pepsin, an anti-PAD2 antibody containing Fab and Fc regions in the above embodiment of the present invention. Alternatively, the Fab' portions below may be bonded via a thioether bond or disulfide bond for the production.

In one embodiment of the present invention, the Fab' antibody refers to an antibody obtained by, for instance, cleaving a disulfide bond in the hinge region of F(ab')₂. The Fab' antibody may be obtained by, for instance, treating F(ab')₂ with a reductant dithiothreitol.

In one embodiment of the present invention, the scFv antibody includes a form of an antibody in which VH and VL are linked via a suitable peptide linker. The scFv antibody may be produced by, for instance, obtaining cDNA encoding a VH and a VL of an anti-PAD2 antibody according to the above embodiment of the present invention, constructing a polynucleotide encoding the VH-peptide linker-VL, cloning the polynucleotide into a vector, and using cells for its expression.

In one embodiment of the present invention, the diabody refers to an antibody having divalent antigen-binding activities and having a form of a scFv dimer. The divalent antigen-binding activities may be the same, or one of the antigen-binding activities may be different from the other. The diabody can be produced by, for instance, constructing a polynucleotide encoding an scFv such that the length of amino acid sequence of its peptide linker is 8 residues or less, cloning the resulting polynucleotide into a vector, and using cells for its expression.

In one embodiment of the present invention, the dsFv refers to an antibody obtained by constructing a polypeptide while a cysteine residue is introduced into each of a VH or a VL and bonding, via a disulfide bond, the above cysteine residues. Where each cysteine residue is introduced may be selected based on an antibody conformation prediction in accordance with the method indicated by Reiter and colleagues (Reiter et al., Protein Eng., 1994 May; 7(5): 697-704).

In one embodiment of the present invention, the antigen-binding peptide or polypeptide refers to an antibody structured by including a VH(s) or a VL(s) of an antibody, or CDRs 1, 2, or 3 thereof. The peptide containing multiple CDRs may be bonded directly or via a suitable peptide linker(s).

The method for producing the above Fv antibody, Fab antibody, F(ab')₂ antibody, Fab' antibody, scFv antibody, diabody, dsFv antibody, or antigen-binding peptide or polypeptide (hereinafter, sometimes referred to as "Fv antibody, etc. ") is not particularly limited. For instance, DNA encoding a region of Fv antibody, etc., in an anti-PAD2 antibody according to the above embodiment of the present invention may be cloned into an expression vector and cells for its expression may be used for the production. Alternatively, a chemical synthesis process such as Fmoc method (fluorenylmethyloxycarbonyl method) or tBOC method (t-butyloxycarbonyl method) may be used for the production. Note that an antigen-binding fragment in the above embodiment of the present invention may be at least one kind of the above Fv antibody, etc.

In one embodiment of the present invention, the anti-PAD2 antibody includes an antibody having heavy and light chains. The heavy chain is typically the main component of a full-length antibody. The heavy chains of a full-length antibody typically have disulfide bonds and non-covalent interactions with the light chains. The heavy chain typically contains a heavy chain variable region (VH) and a constant region. The light chain is typically a component of a full-length antibody and is distinct from the heavy chain. The light chain typically contains a light chain variable region (VH) and a constant region.

In one embodiment of the present invention, each CDR (complementarity determining region) is a region that forms a site of binding to its antigen in the antibody. Typically, the CDR is located on the Fv (variable region) of the antibody. Typically, there are heavy-chain CDR1, CDR2, and CDR3, and light-chain CDR1, CDR2, and CDR3 (sometimes referred to as HCDR1, HCDR2, HCDR3, LCDR1, LCDR2, and LCDR3, respectively). Typically, each CDR has about 3 to 30 amino acid residues. It is known that the CDRs of the heavy chain contribute particularly to the binding of the antibody to the antigen. Among the CDRs, CDR3 is known to contribute the most to the binding of the antibody to the antigen. The Fv regions other than CDRs are called framework regions and include FR1, FR2, FR3 and FR4, which are relatively well conserved among antibodies (e.g., Kindt et al., Kuby Immunology, 6th ed., W.H. Freeman and Co.)

In one embodiment of the present invention, the anti-PAD2 antibody includes an anti-PAD2 antibody having HCDR1, HCDR2, HCDR3, LCDR1, LCDR2, or LCDR3, wherein HCDR1 may contain the amino acid sequence represented by SEQ ID NO: 3, 9, or 15, HCDR2 may contain the amino acid sequence represented by SEQ ID NO: 4, 10, or 16, HCDR3 may contain the amino acid sequence represented by SEQ ID NO: 5, 11, or 17, LCDR1 may contain the amino acid sequence represented by SEQ ID NO: 6, 12, or 18 LCDR2 may contain the amino acid sequence represented by SEQ ID NO: 7, 13, or 19, or LCDR3 may contain the amino acid sequence represented by SEQ ID NO: 8, 14, or 20. In one embodiment of the present invention, the anti-PAD2 antibody includes an anti-PAD2 antibody comprising: (a) HCDR1 containing the amino acid sequence represented by SEQ ID NO: 3, HCDR2 containing the amino acid sequence represented by SEQ ID NO: 4, HCDR3 containing the amino acid sequence represented by SEQ ID NO: 5, LCDR1 containing the amino acid sequence represented by SEQ ID NO: 6, LCDR2 containing the amino acid sequence represented by SEQ ID NO: 7, and LCDR3 containing the amino acid sequence represented by SEQ ID NO: 8; (b) HCDR1 containing the amino acid sequence represented by SEQ ID NO: 9, HCDR2 containing the amino acid sequence represented by SEQ ID NO: 10, HCDR3 containing the amino acid sequence represented by SEQ ID NO: 11, LCDR1 containing the amino acid sequence represented by SEQ ID NO: 12, LCDR2 containing the amino acid sequence represented by SEQ ID NO: 13, and LCDR3 containing the amino acid sequence represented by SEQ ID NO: 14; or (c) HCDR1 containing the amino acid sequence represented by SEQ ID NO: 15, HCDR2 containing the amino acid sequence represented by SEQ ID NO: 16, HCDR3 containing the amino acid sequence represented by SEQ ID NO: 17, LCDR1 containing the amino acid sequence represented by SEQ ID NO: 18, LCDR2 containing the amino acid sequence represented by SEQ ID NO: 19, and LCDR3 containing the amino acid sequence represented by SEQ ID NO: 20, (herein referred to as any of CDR sets (a)-(c) for short).

In one embodiment of the present invention, the CDR(s) may be defined by Kabat's definition (Sequences of Proteins of Immunological Interest, 5th ed. Bethesda, MD.(1991)), IMGT definition (Lefranc et al., Dev Comp Immunol., 2003 Jan;27(1):55-77.), or Chothia's definition (Chothia et al., J. Mol. Biol., 1987;196:901-917). In one embodiment of the present invention, the CDR(s) is defined preferably by Kabat's definition.

In one embodiment of the present invention, the anti-PAD2 antibody includes an antibody having heavy and light chain variable regions. Here, the heavy chain variable region may contain the amino acid sequence represented by SEQ ID NO: 21, 23, or 25, and the light chain variable region may contain the amino acid sequence represented by SEQ ID NO: 22, 24, or 26. In one embodiment of the present invention, the anti-PAD2 antibody comprises (d) a heavy chain variable region and a light chain variable region containing the amino acid sequences set forth in SEQ ID NOs: 21 and 22, respectively, (e) a heavy chain variable region and a light chain variable region containing the amino acid sequences set forth in SEQ ID NOs: 23 and 24, respectively, or (f) a heavy chain variable region and a light chain variable region containing the amino acid sequences set forth in SEQ ID NOs: 25 and 26, respectively (herein referred to as any of the variable region sets (d)-(f) for short).

In one embodiment of the present invention, the anti-PAD2 antibody includes an antibody having heavy and light chain constant regions. Here, the heavy chain constant region may contain the amino acid sequence represented by SEQ ID NO: 27, and the light chain constant region may contain the amino acid sequence represented by SEQ ID NO: 28.

In Example 1 below, the nucleotide and amino acid sequences of each obtained antibody were examined by DNA sequencing. The results have revealed that the amino acid sequences of the CDRs of PK1-16 in Example 1 were HCDR1:SYAMY (SEQ ID NO: 3), HCDR2:GISSSGRYTGYAPAVKG (SEQ ID NO: 4), HCDR3:DVYDSWTYANRIDA (SEQ ID NO: 5), LCDR1:SGGGRRGYYG (SEQ ID NO: 6), LCDR2:NNDERPS (SEQ ID NO: 7), and LCDR3:GSGDTTTDSGI (SEQ ID NO: 8). In addition, the amino acid sequences of the CDRs of CK1-10 in Example 1 were HCDR1:DYGMG (SEQ ID NO: 9), HCDR2:AISNRGSHTYYGAAVKG (SEQ ID NO: 10), HCDR3:DAGTCISSYGFSCVSAASIDA (SEQ ID NO: 11), LCDR1: SGGSGSYGGSYYYG (SEQ ID NO: 12), LCDR2:DNTNRPS (SEQ ID NO: 13), and LCDR3:GSIDSISDADI (SEQ ID NO: 14). Further, the amino acid sequences of the CDRs of CK1-14 in Example 1 were HCDR1:RYAIQ (SEQ ID NO: 15), HCDR2:VINSGGRTLYYAPAVKG (SEQ ID NO: 16), HCDR3:GGYAYGIET (SEQ ID NO: 17), LCDR1:SGSRYDYG (SEQ ID NO: 18), LCDR2 :YNNKRPS (SEQ ID NO: 19), and LCDR3:GSTDTSNDI (SEQ ID NO: 20). These CDR sequences of PK1-16, CK1-10 and CK1-14 are all defined by Kabat's definition. Here, the amino acid sequences of the variable regions of PK1-16 were
VH:AVTLDESGGGLQTPGGGLSLUCKASGFTFRSYAMYWVRQAPGKGLEWLAGISSSGRYTGYAPAV KGRATISRDNGQSTVRLQLSNLRAEDAGTYYCAK DVYDSWTYANRIDAWGHGTEVIVSS (SEQ ID NO: 21) and
VL:ALTQPSVSANPGETVKITCCSGGGRRRGYYGWYQQKSPGSAPVTVIYNNDERPSNIPSRFSGFKSG STATLTITGVQAEDEA VYYCGSGDTTTDSGIFGAGTTLTVL (SEQ ID NO: 22). In addition, the amino acid sequences of the variable regions of CK1-10 were
VH:AVTLDESGGGLQTPGRALSLVCKASGFTFSDYGMGWMRQAPGKGLEWVGAISNRGSHTYYYGA AVKGRATISRDNGQSTVRLQLNNLRAEDTGTYYCAKDAGTCI SYGFSCVSAASIDAWGHGTEVIVSS (SEQ ID NO: 23) and
VL:ALTQPSSVSANLGGTVKITCCSGGSGSYGGSYYYGWYQQKAPGSAPVTLIYDNTNRPSNIPSRFSG SKSGSTATLTITGVQADDEA VYFCGSIDSISDADIFGAGTTLTVL (SEQ ID NO: 24). Further, the amino acid sequences of the variable region of CK1-14 were
VH:AVTLDESGGGLQTPGGALSLVCKASGFTFTRYAIQWVRQAPGKGLEWVGVINSGGRTLYAPAVKG RATISRDNGQSTVRLQLNNLRAEDTAI YYCVRGGYAYGIETWGHGTEVIVSS (SEQ ID NO: 25) and VL:ALTQPSVSANPGETVKITCSGSRYDYGWYQQKSPGSAPVTLIYYNNKRPSDIPSRFSGSKSGSTHTL TITGVQADD EAVYFCGSTDTSNDIFGAGTTLTVL (SEQ ID NO: 26). The nucleotide sequences corresponding to the amino acid sequences represented by SEQ ID NOs: 3 to 26 were the nucleotide sequences set forth in SEQ ID NOs: 29 to 52, respectively.

In Example 1 below, expression vectors were used for antibody expression. According to the results, the heavy-chain and light-chain constant regions of PK1-16, CK1-10, or CK1-14 were the amino acid sequences represented by SEQ ID NOs: 27 and 28, respectively. The nucleotide sequences corresponding to the amino acid sequences represented by SEQ ID NOs: 27 and 28 were those set forth in SEQ ID NOs: 53 and 54, respectively.

In one embodiment of the present invention, the anti-PAD2 antibody includes an anti-PAD2 antibody that competes with an antibody (e.g., an antibody having any of the CDR sets of (a)-(c) above) (hereinafter, sometimes referred to as a reference antibody) according to an embodiment of the present invention for binding to PAD2. The competition involves the binding of an antibody to its antigen thereby inhibiting the binding of another antibody to the same antigen. The competing antibody can be checked, for example, by a competitive binding assay. Examples of the competitive binding assay include competitive ELISA or competitive FACS analysis (see, for example, Zhou et al., J Gen Virol., 2008 Feb; 89(Pt 2): 500-508.). The binding may be measured, for example, by surface plasmon resonance. A competitive binding assay may include, for example, the steps of: coating an antigen on a microplate, adding a test antibody and incubating them to form binding between the antigen and the test antibody; adding a labeled reference antibody to wells, incubating and washing it; or quantifying the amount of binding of the labeled reference antibody to the antigen. At this time, for example, the amount of binding of the biotinylated reference antibody may be detected by measuring absorbance at the measurement wavelength of 450 nm by using HRP-conjugated streptavidin and 3,3',5,5'-tetramethylbenzidine. The inhibition rate may be evaluated as the percentage of decrease in the amount of binding of the labeled reference antibody when compared to the absence of the test antibody or the addition of a negative control. Inhibition of the reference antibody by the competing antibody includes inhibition of 20, 30, 40, 50, 60, 70, 80, 90% or more, or 100%. This inhibition is preferably 40% or higher, and particularly preferably 50% or higher. The competing antibody may have binding affinity to the epitope to which the reference antibody binds.

One embodiment of the present invention is a polynucleotide or vector encoding an anti-PAD2 antibody (e.g., an antibody having any of the CDR sets in (a)-(c) above) according to the above embodiment of the present invention. This polynucleotide or vector may be introduced into a cell to produce a transformant. The transformant may be a human cell or a non-human mammalian (e.g., rat, mouse guinea pig, rabbit, cow, monkey) cell. Examples of the mammalian cell include Chinese hamster ovary cells (CHO cells), monkey cell COS-7, or human embryonic kidney cells (e.g., HEK293 cells). Alternatively, the transformant may be *Escherichia coli,* yeast, or the like. The above polynucleotide or vector may be constructed to be able to express an anti-PAD2 antibody. The above polynucleotide or vector may contain, for instance, elements necessary for protein expression, such as a promoter, an enhancer, a replication origin, and/or an antibiotic resistance gene.

Examples of the above vector that can be used include *E. coli* plasmids (e.g., pET-Blue), *Bacillus subtilis* plasmids (e.g., pUB110), yeast plasmids (e.g., pSH19), expression plasmids for animal cells (e.g., pA1-11, pcDNA3.1-V5/His-TOPO), bacteriophages such as λ phage, or viral vectors. The vector may be an expression vector or linear.

Examples of an available method for introducing the above polynucleotide or vector into a cell include a calcium phosphate method, lipofection, electroporation, an adenoviral method, a retroviral method, or microinjection (the revised fourth ed., New Gene Engineering Handbook, YODOSHA CO., LTD., (2003): 152-179). Examples of an available method for producing an antibody by using a cell include a method described in "Protein Experiment handbook, YODOSHA CO., LTD., (2003); 128-142)".

One embodiment of the present invention is a method for producing an anti-PAD2 antibody, comprising the step of growing a cell comprising the polynucleotide or vector according to the above embodiment of the present invention. The above growing step includes a culturing step. In addition, this production method may include a step of collecting the anti-PAD2 antibody. Further, this production method may include a step of preparing a cell culture medium. Furthermore, this production method may include a step of purifying the anti-PAD2 antibody.

In one embodiment of the present invention, for purification of the antibody, it is possible to use, for instance, ammonium sulfate or ethanol precipitation, Protein A, Protein G, or gel filtration chromatography, anion or cation exchange chromatography, phosphocellulose chromatography, hydrophobic interaction chromatography, affinity chromatography, hydroxyapatite chromatography, or lectin chromatography (Protein Experiment handbook, YODOSHA CO., LTD., (2003): 27-52).

One embodiment of the present invention is a composition comprising an anti-PAD2 antibody. This composition can be used to inhibit fibrosis. In one embodiment of the present invention, examples of the composition include a pharmaceutical composition. Examples of the composition include a composition for the treatment of fibrotic disease. This composition optionally contains at least one pharmaceutically acceptable carrier. Also, the form of the carrier is not particularly limited, and may be, for instance, a solid or liquid. The carrier may be, for example, a buffer or excipient. The content of the carrier may be, for instance, a pharmaceutically effective amount. The effective amount may be, for instance, an amount sufficient to pharmaceutically stabilize or deliver the active ingredient. For instance, the buffer is effective in stabilization of the active ingredient in a vial. It is also preferable to contain a therapeutically effective amount or effective amount of the active ingredient so as to elicit a desired effect (e.g., fibrosis-inhibiting effect). Here, a dosage form of the pharmaceutical composition is not limited as long as it can be used for treatment, and may be an active ingredient alone or may be a mixture of an active ingredient and any component(s). Also, the form of the composition is not particularly limited, and may be, for instance, a liquid or lyophilized preparation. The anti-PAD2 antibody contained in the composition may be a PAD2-neutralizing antibody as described above.

One embodiment of the present invention is a pharmaceutical composition for treating fibrotic disease, comprising an antibody that specifically binds to PAD2, and a pharmaceutically acceptable carrier. This pharmaceutical composition can be used to treat fibrotic disease.

One embodiment of the present invention is a method of inhibiting or treating fibrosis in a subject in need thereof, comprising the step of administering to the subject an effective amount of a pharmaceutical composition comprising an antibody that specifically binds to PAD2, and a pharmaceutically acceptable carrier. This method can be used to inhibit or treat fibrosis. One embodiment of the present invention is a pharmaceutical composition used to inhibit or treat fibrosis in a subject in need thereof, comprising an antibody that specifically binds to PAD2, and a pharmaceutically acceptable carrier. This pharmaceutical composition can be used to inhibit or treat fibrosis. One embodiment of the present invention is use of an anti-PAD2 antibody for the manufacture of a pharmaceutical composition for inhibiting or treating fibrosis. The anti-PAD2 antibody used in the method, pharmaceutical composition, or use may be a PAD2-neutralizing antibody as described above.

One embodiment of the present invention is a composition comprising an anti-PAD2 antibody (e.g., an antibody having any of the CDR sets in (a)-(c) above) according to the above embodiment of the present invention, wherein the composition is used to inhibit PAD2 activity. One embodiment of the present invention is a method of inhibiting PAD2 activity, comprising the step of bringing PAD2 into contact with the anti-PAD2 antibody according to the above embodiment of the present invention.

One embodiment of the present invention is a kit comprising an anti-PAD2 antibody. This kit can be used to inhibit fibrosis. Examples of the kit include a kit for inhibiting or treating fibrosis. This kit may contain, for example, the above composition and may also contain a package insert, a buffer solution, a container, or packaging.

In one embodiment of the present invention, the treatment involves optionally exerting an effect of ameliorating, alleviating, inhibiting, suppressing relapse of, or preventing at least one symptom of a subject. In one embodiment of the present invention, the treatment of fibrosis includes inhibiting fibrosis. In one embodiment of the present invention, the inhibition of fibrosis can result in treatment of fibrotic disease, treatment of a disease with fibrosis, or treatment of a disease caused by fibrosis. In one embodiment of the present invention, the disease with fibrosis includes, for example, cirrhosis (e.g., liver cirrhosis).

The route of administration of the antibody or composition to a subject should be effective for the treatment, and may be, for example, intravenous, subcutaneous, intramuscular, or intraperitoneal. The dosage form should be effective for treatment, and may be, for instance, an injection. An aqueous solution for injection may be stored, for example, in a vial or stainless steel container. In addition, the aqueous solution for injection may also contain, for example, saline, sugar (e.g., trehalose), NaCl, or NaOH. Also, the composition may also be blended with effective amounts of, for instance, a buffer (e.g., a phosphate buffer), a pH modifier, and/or a stabilizer.

The dose, dosing interval, or administration method of the antibody or composition may be selected, if appropriate, in view of the age, body weight, symptom, and/or affected organ of a patient. The dose may be, for example, 0.01 to 200 mg/kg body weight per administration. The dosing interval, for example, may be 1 or 2 doses every 1 to 28 days.

In one embodiment of the present invention, examples of the subject (including a patient) include a human or a non-human mammal (e.g., at least one species of a mouse, a guinea pig, a hamster, a rat, a mouse, a rabbit, a pig, sheep, a goat, a cow, a horse, a cat, a dog, a marmoset, a monkey, or a chimpanzee). Meanwhile, the patient may also be a patient diagnosed as having fibrosis or in need of treatment.

In one embodiment of the present invention, the effect of inhibiting or treating fibrosis may be evaluated, for example, by measuring the level of decrease in the amount or percentage of fibrosis tissue in an individual or tissue after administration of an anti-PAD2 antibody. The effect of inhibiting or treating fibrosis may be evaluated by pathological diagnosis. In the pathological diagnosis, collected biopsy tissue sections may be stained. The staining may be performed, for example, using the Sirius Red staining method. Further, the amount or percentage of fibrosis tissue may be calculated by image analysis of the staining results. If the amount or percentage of fibrosis tissue after the anti-PAD2 antibody administration is significantly lower than that before the administration or the negative control administration, it may be judged that the inhibition or treatment is effective. In one embodiment of the present invention, the effect of inhibiting or treating fibrosis may be measured, for example, by using, as an indicator, the level of fibrosis marker(s) (e.g., α-SMA) in a subject or subject-derived sample. At this time, if the level of marker after the anti-PAD2 antibody administration is significantly lower than that before the antibody administration or the negative control administration, it may be judged that the inhibition or treatment is effective.

In one embodiment of the present invention, the effect of inhibiting PAD2 activity may be measured, for example, by using, as an indicator, the amount of citrullinated compound(s) (e.g., a protein or peptide) in a subject or subject-derived sample. At this time, when the amount of citrullinated compound(s) after the anti-PAD2 antibody treatment is significantly lower than that before the antibody treatment or the negative control treatment, the inhibition may be judged to be effective. In addition, if the amount of citrullinated compound(s) after the anti-PAD2 antibody treatment decreases to 90, 70, 50, 30, or 10% or less, or 0%, when compared to the amount before the antibody treatment or the amount at the negative control treatment, the inhibition may be judged to be effective.

One embodiment of the present invention is a composition comprising an antibody that specifically binds to PAD2 extracellularly. This composition can be used to inhibit fibrosis as demonstrated in the Examples below. In one embodiment of the present invention, the anti-PAD2 antibody includes an antibody that inhibits PAD2 activity extracellularly or an antibody present extracellularly. Examples of the anti-PAD2 antibody include an antibody that is present extracellularly and is absent intracellularly after administration to a subject. Examples of the anti-PAD2 antibody include an antibody that inhibits PAD2 activity extracellularly and does not inhibit the PAD2 activity intracellularly after administration to a subject. Examples of the anti-PAD2 antibody include an antibody that inhibits extracellular substrate citrullination after administration to a subject but not intracellular substrate citrullination.

In one embodiment of the present invention, the amino acid is a general term for any organic compound having an amino group and a carboxyl group. When an antibody according to an embodiment of the present invention contains a "specific amino acid sequence", any of amino acids in the amino acid sequence may be chemically modified. In addition, any of amino acids in the amino acid sequence may form a salt or a solvate. In addition, any of amino acids in the amino acid sequence may be in an L-form or D-form. In such cases, an antibody according to an embodiment of the present invention can be said to contain the above "specific amino acid sequence". Examples of the *in vivo* chemical modification of amino acids included in a protein include N-terminal modification (e.g., acetylation, myristoylation), C-terminal modification (e.g., amidation, glycosylphosphatidylinositol addition), or side-chain modification (e.g., phosphorylation, glycosylation).

In one embodiment of the present invention, the binding may be mediated by either a covalent bond or noncovalent bond, and may involve, for instance, an ion bond, a hydrogen bond, a hydrophobic interaction, or a hydrophilic interaction.

In one embodiment of the present invention, the term "significant(ly)" may mean a state of p < 0.001, p < 0.05, or p < 0.01 after a statistically significant difference is evaluated using Student's t-test (one- or two-tailed) or Bonferroni multiple comparison test. Alternatively, the term may refer to a state where a substantial difference occurs.

All the literatures cited herein are incorporated by reference in their entirety. In one embodiment of the present invention, the term "or" is used when "at least one" matter listed in the text is acceptable. The same applies to "or". When the wording "number between any two" is indicated herein, this range encompasses the two numbers inclusive. As used herein, the wording "from A to B" herein means A or more and B or less.

Hereinabove, embodiments of the present invention have been described. However, they are examples of the present invention. Hence, various configurations other than the above can be adopted. In addition, the configurations described in the above embodiments may be combined and adopted.

### Examples

Hereinbelow, the present invention will be described in more detail with reference to Examples. However, the present invention is not limited to them.

### Example 1: To produce anti-PAD2 antibody

Three 3-month-old Boris Brown chickens were each intraperitoneally immunized with 333 µg of a KLH-conjugated peptide antigen (SEQ ID NO: 2; positions 341 to 357 of PAD2). Complete Freund's adjuvant (014-09541, Wako) for primary immunization and incomplete Freund's adjuvant (011-09551, Wako) for secondary or tertiary immunization were used with the antigen for immunization. For quaternary immunization, the antigen diluted with PBS (phosphate buffered saline) was intravenously injected. Blood was collected from the wing vein every other week, and the antibody titer was checked by ELISA. After the tertiary immunization, the quaternary immunization was conducted as the final immunization. Three days after the final immunization, the spleen of each chicken was removed; lymphocytes were isolated by density gradient centrifugation using Ficoll-Paque PLUS (17-1440-03, Cytiva); and a TRIzole reagent (15596026, Life Technologies) was used to extract RNA. The extracted RNA was subjected to RT-PCR using a Prime Script II 1st Strand cDNA Synthesis Kit (6210A, TAKARA) to synthesize cDNA. Then, an scFv phage library was prepared. The expression vector used was a pPDS vector. The scFv phage library was prepared in accordance with the method described in a reference document: "Nakamura et al., J Vet Med Sci., 2004 Ju;66 (7): 807-814".

The scFv phage antibody library was used to perform panning using a plate on which the full-length PAD2 had been immobilized. The panning was performed in accordance with the method described in a reference document: "Nakamura et al., J Vet Med Sci., 2004 Ju;66 (7): 807-814". After the fifth panning, the reactivity of the library was checked by ELISA using a plate on which a synthetic peptide was immobilized. A phage screening was conducted in the library with increased reactivity. During the screening, *E. coli* cells were infected with their phages, and plated on a 2×YT Agar plate(s) containing ampicillin (50 µg/ml, NACALAI TESQUE, INC.). Each of the resulting colonies was cultured in 2×YT liquid medium containing ampicillin. After infection with a helper phage, phage induction was carried out in 2×YT liquid medium containing ampicillin (50 µg/ml), kanamycin (25 µg/ml, Meiji Seika, Inc.), and IPTG (100 µg/ml, NACALAI TESQUE, INC.). The reactivity of the resulting scFv phage antibody in the culture supernatant was checked by ELISA using an antigen-immobilized plate. The positive clones obtained were sequenced to determine the sequences.

Clones with different sequences were each subjected to PCR while the DNA strand encoding each scFv antibody was used as a template to amplify the H-chain variable region and the L-chain variable region of the chicken-derived antibody gene. The amplified fragments were each inserted by homologous recombination using Seamless Cloning and Assembly Enzyme Mix (Thermo, A14606) into a preconstructed expression vector containing the human H-chain constant region (IgG1) and the human L-chain constant region. The H-chain and L-chain constructs produced were transfected into 293 cells. After that, the reactivity was checked by ELISA using the full-length PAD2 immobilized. Of the resulting antibody clones, PK1-16, CK1-10, and CK1-14 were used in the following experiments.

### Example 2: Human PAD2-binding affinity of anti-PAD2 antibody

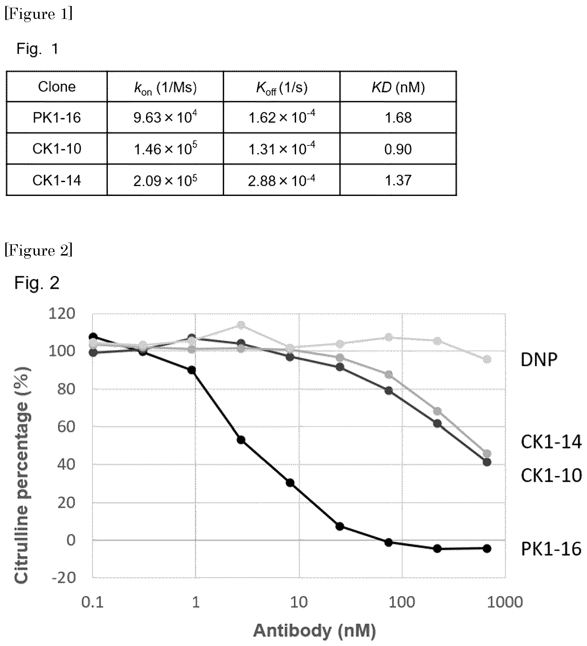

To evaluate the binding affinity of each anti-PAD2 antibody for human PAD2 protein, the dissociation constant (K_{D}) was measured using a surface plasmon resonance system Biacore 8K (Cytiva). Series S Sensor Chip Protein A (Cytiva) was used as the sensor chip. HBS-EP+ buffer was used as the running buffer. The test antibody was prepared at 0.8 µg/mL by using the running buffer to make a ligand solution. The ligand solution was added to the flow cell at a flow rate of 10 µL/min for 60 seconds, and the flow cell without the ligand solution was used as a reference cell. The running buffer was used to prepare human PAD2 protein from several hundred pM to several tens of nM to make each analyte solution. The running buffer was also used as a blank solution. The blank solution or analyte solution was added at a flow rate of 30 µL/min for 120 seconds by using the single-cycle method, and the dissociation time was set to 1200 seconds. The sensorgram of the reference cell was subtracted from the sensorgram of the flow cell with the ligand when the blank solution or analyte solution was added. In addition, the sensorgram when the blank solution was added was subtracted from the sensorgram when the analyte solution was added, and then used for analysis. Biacore^{™} Insight Evaluation Software (Cytiva) was used to calculate binding parameters while using the 1:1 binding model. Fig. 1 shows the results. The anti-PAD2 antibodies showed favorable affinity.

### Example 3: Inhibition of PAD2 activity by anti-PAD2 antibody

The ability of each anti-PAD2 antibody to inhibit human PAD2 activity was evaluated. Human PAD2 (the final concentration: 10 nM) was admixed with 20 mM Tris buffer (pH 7.6) containing 1 mM DTT, 150 mM NaCl, and 0.3 mM CaCl₂ so that the final concentrations of the anti-PAD2 antibody were 600, 200, 66.67, 22.22, 7.41, 2.47, 0.82, 0.27, and 0.09 nM. After incubation at 37°C for 1 hour, BAEE (benzoylarginine ethyl ester) was added with stirring and the mixture was stirred well (the final concentration of BAEE was 10 mM). After incubation of the solution at 37°C for 3 hours, the citrulline residues of the citrullinated BAEE were colorimetrically quantified using a mixture containing 2,3-butanedione monoxime and thiosemicarbazide. Anti-dinitrophenyl (DNP) antibody was used as a control. Fig. 2 shows the results. Each anti-PAD2 antibody inhibited human PAD2 activity in a concentration-dependent manner.

### Example 4: Antifibrosis effect of anti-PAD2 antibody in fibrosis model

### 4.1 Production of fibrosis model and administration of anti-PAD2 antibody

The drug efficacy of each anti-PAD2 antibody was evaluated using a fibrosis model. First, bile duct ligation-induced cholestatic liver disease (BDL) model mice were generated and treated with each anti-PAD2 antibody according to the following procedure. Seven-week-old male C57BL/6J mice were each anesthetized by intraperitoneal administration of a triad anesthesia (medetomidine hydrochloride/mitazolam/butorphanol tartrate) at 0.75/4/5 mg/kg. After confirming that the animals were each anesthetized, a hair clipper was used to clip the hair around the abdominal xiphoid process of the animal. The clipped area was disinfected and the abdomen was opened about 1 cm. A cotton swab, for instance, was used to expose the bile ducts in the duodenal portion. The common bile duct was ligated at two sites with silk suture 7-0 with a sterile Nesco Suture needle. After ligation, the bile duct was not cut, the bile duct was returned to its original position, and the peritoneum was sutured with silk suture 3-0 with a Nesco Suture sterile needle. The epidermis was sutured with an automatic suture machine loaded with suture clips. Atipamezole hydrochloride was administered intraperitoneally at 0.75 mg/kg, and the mice were awakened and returned to the cage. The mice were maintained on a warming pad until fully awake from anesthesia. One hour after surgery, on days 4, 7, and 11, each anti-PAD2 antibody or control antibody (anti-DNP antibody) was administered intravenously (at 25 mg/kg). The sham-operated (mice with open chest but without bile duct ligation) group was used as the control group. Ten model mice in the control group, five in the anti-DNP antibody group, and seven in each of the anti-PAD2 antibody groups were used for the final analysis.

### 4.2 To measure fibrosis percentage

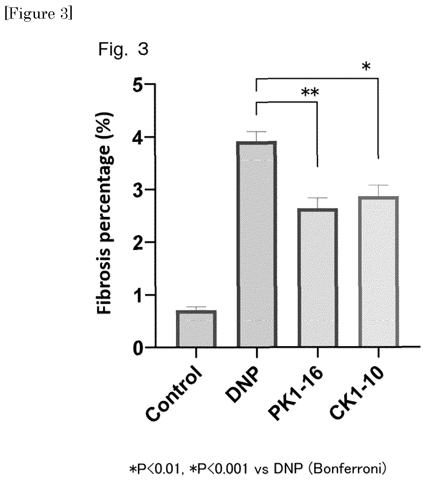

The liver tissue collected on day 13 after surgery was immersed in Bouin's fixative solution, fixed at room temperature for 24 hours, and then paraffin-embedded. Paraffin sections were prepared, deparaffinized and hydrophilized with xylene, 100% to 70% alcohol series, and RO water, and then immersed in 0.03% Picro-Sirius Red solution (Fujifilm Wako Pure Chemicals Corporation) for 60 minutes. After passing through 0.5% acetic acid solution and RO water, stained sections were dehydrated and permeabilized with 70% to 100% alcohol series and xylene, and then sealed with Entellan new (Merck) for observation. Specimens were photographed using a bright-field microscope with a CCD camera (Leica Microsystems) at 100x field of view for five views per section. Based on the images taken, the area of each field of view and the Sirius Red positive area were measured, and fibrosis percentage (fibrotic area) was calculated using ImageJ software (National Institute of Health). Fig. 3 shows the results. Each anti-PAD2 antibody was administered to significantly inhibit fibrosis.

### 4.3 To measure ability to inhibit PAD2 activity

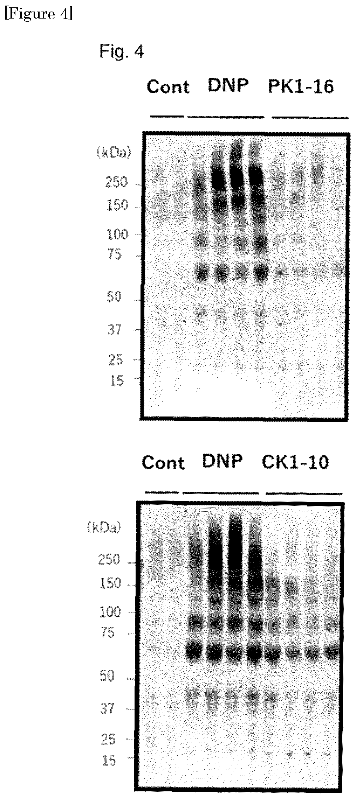

On day 13 after surgery, the liver was collected and proteins in the tissue were extracted with RIPA buffer. Equal amounts of total protein (50 µg) were used, separated by sodium dodecyl sulfate-polyacrylamide gel electrophoresis (SDS-PAGE), and electrically transferred onto a PVDF membrane. The membrane was incubated with a primary antibody (Anti-peptidyl-citrulline, clone F95 Antibody; Sigma-Aldrich). Next, the membrane was incubated with a secondary antibody conjugated with horseradish peroxidase (Goat anti-Mouse IgM Secondary Antibody HRP; Novus biologicals). Finally, citrullinated proteins were detected by chemiluminescence (ECL&trade Prime Western Blotting Detection Reagent; Cytiva). Fig. 4 shows the results. Each anti-PAD2 antibody was administered to inhibit PAD2 activity, thereby preventing an increase in the amount of citrullinated proteins.

As described in the above Examples, the experiments have revealed that the anti-PAD2 antibodies inhibit fibrosis.

Hereinabove, the present invention has been described based on the Examples. The Examples are just examples. Those skilled in the art should understand that various modifications are allowed and such modified embodiments are within the scope of the present invention.

Sequence Listing

## Claims

1. A composition for inhibiting fibrosis, comprising an antibody that specifically binds to PAD2 (Peptidylarginine deiminase 2).

2. The composition according to claim 1, wherein the antibody is a PAD2-neutralizing antibody.

3. The composition according to claim 1 or 2, wherein the antibody is an antibody that inhibits fibrosis.

4. The composition according to any one of claims 1 to 3, wherein the antibody is an antibody that binds to the PAD2 extracellularly.

5. The composition according to any one of claims 1 to 4, wherein the antibody is a monoclonal antibody.

6. The composition according to any one of claims 1 to 5, wherein the antibody has a K_{D} (M) toward the PAD2 of 9.0 × 10⁻⁹ or less.

7. The composition according to any one of claims 1 to 6, wherein the antibody is an antigen-binding fragment.

8. A pharmaceutical composition for treating fibrotic disease, comprising an antibody that specifically binds to PAD2 and a pharmaceutically acceptable carrier.

9. A kit for inhibiting fibrosis, comprising an antibody that specifically binds to PAD2.
